(19) **Europäisches Patentamt**
**European Patent Office**
**Office européen des brevets**

(11) **EP 2 742 863 B1**

(12) **EUROPEAN PATENT SPECIFICATION**

(45) Date of publication and mention
of the grant of the patent:
**14.06.2017 Bulletin 2017/24**

(51) Int Cl.:
*A61B 6/00* (2006.01)  *G01T 7/00* (2006.01)
*H01L 27/146* (2006.01)  *H01L 31/09* (2006.01)
*H04N 5/32* (2006.01)  *G01T 1/24* (2006.01)
*H04N 5/335* (2011.01)  *G06T 5/20* (2006.01)
*G06T 3/20* (2006.01)  *H04N 7/01* (2006.01)

(21) Application number: **12807470.5**

(22) Date of filing: **09.08.2012**

(86) International application number:
**PCT/JP2012/070374**

(87) International publication number:
**WO 2013/005863 (10.01.2013 Gazette 2013/02)**

(54) **RADIOGRAPH IMAGING DEVICE AND RADIOGRAPH IMAGING METHOD**

RADIOGRAPHISCHE ABBILDUNGSVORRICHTUNG UND RADIOGRAPHISCHES
ABBILDUNGSVERFAHREN

DISPOSITIF D'IMAGERIE PAR RADIOGRAPHIE ET PROCÉDÉ D'IMAGERIE PAR RADIOGRAPHIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.08.2011 JP 2011177362**

(43) Date of publication of application:
**18.06.2014 Bulletin 2014/25**

(73) Proprietor: **Fujifilm Corporation
Minato-ku
Tokyo 106-8620 (JP)**

(72) Inventors:
• **OKADA, Yoshihiro
Ashigarakami-gun
Kanagawa 258-0023 (JP)**
• **KUWABARA, Takao
Ashigarakami-gun
Kanagawa 258-0023 (JP)**

(74) Representative: **Klunker . Schmitt-Nilson . Hirsch
Patentanwälte
Destouchesstraße 68
80796 München (DE)**

(56) References cited:
**JP-A- 2000 244 733    JP-A- 2002 350 547
JP-A- 2003 255 049    JP-A- 2007 059 887
JP-A- 2009 021 988    JP-A- 2009 049 562**

• **VAN DE VILLE D ET AL: "Image resampling
between orthogonal and hexagonal lattices",
INTERNATIONAL CONFERENCE ON IMAGE
PROCESSING (ICIP), IEEE, vol. 3, 22 September
2002 (2002-09-22), pages 389-392, XP010607736,
ISBN: 978-0-7803-7622-9**
• **KEMNA A ET AL: "Low noise, large area CMOS
x-ray image sensor for C.T. application",
PROCEEDINGS OF IEEE SENSORS 2003 (IEEE
CAT. NO.03CH37498) IEEE PISCATAWAY, NJ,
USA; [IEEE INTERNATIONAL CONFERENCE ON
SENSORS], IEEE, vol. CONF. 2, 22 October 2003
(2003-10-22) , page 1260, XP010690979, DOI:
10.1109/ICSENS.2003.1279147 ISBN:
978-0-7803-8133-9**

**Description**

Technical Field

The present invention relates to a radiographic imaging device and a radiographic imaging method for imaging radiographic images.

Background Art

Recently radiographic imaging devices that employ a radiation detection element such as a FPD (flat panel detector) that has an X-ray sensitive layer disposed on a TFT (Thin Film Transistor) active matrix substrate and is capable of directly converting X-ray information into digital data, have been put into practice. Such FPDs have the advantage that images can be checked more immediately than with conventional imaging plates, and that video images can also be checked, hence the introduction of FPDs is proceeding rapidly. Various types are proposed for such radiation detection elements, and there are, for example, direct-conversion-type radiation detection elements that convert radiation directly to charge in a semiconductor layer, and accumulate the charge, and also indirect-conversion-type radiation detection elements that first convert radiation into light with a scintillator, such as CsI:Tl, GOS (Gd$_2$O$_2$S:Tb), then convert the converted light into charge in a semiconductor layer, and accumulate the charge.

In radiation detection element, for example, plural scan lines and plural signal lines are disposed so as to intersect with each other, and pixels corresponding to each of the intersections between the scan lines and the signal line are provided in a matrix pattern. The plural scan lines and signal lines are connected to external circuits (for example an amplification integrated circuit (IC) and gate IC) at peripheral portions of the radiation detection element.

Making the size of the pixels of the radiation detection element smaller is effective in raising the resolution of a FPD. Particularly in direct-conversion-type radiation detection elements that employ for example Se or the like, since the pixel size directly contributes to raising the resolution, there are various proposals for radiation detection elements that raise image quality by miniaturization. For example, there is a proposal for a product with small pixel size for an FPD for use in mammography that has an emphasis on resolution.

However, the charge amount capable of being collected reduces in proportion to the reduction in pixel size, and as a result the sensitivity (S/N) is lowered. There are accordingly sometimes cases in which even though the resolution is raise, the detective quantum efficiency (DQE: proportional to {S/N × 1/ resolution}) actually drops.

However, there is a proposal for a detection device to realize an increase in both resolution and sensitivity in which pixels are disposed in the X/Y directions staggered with respect to each other by half the pitch, and pixel interpolation processing is performed based on the generated image data (see, for example, Japanese Patent Application Laid-Open (JP-A) No. 2003-255049). There is also a proposal for an X-ray detection device that employs hexagonal shaped pixels to raise the light utilization ratio (see, for example, JP-A No. 2006-29839).

For example, the length of the longest diagonal of a regular hexagonal shape (maximum diagonal length) d1max and a surface area S1 exhibit the following relationship.

$$\mathrm{d1max} = \sqrt{((8/3\sqrt{3}) \times S1)} \approx \sqrt{(1.54 \times S1)}$$

In cases in which the regular hexagonal shape surface area S1 = 10000 $\mu$m$^2$, a comparison between regular hexagonal shaped pixels and square shaped pixels having the same pixel surface area shows that:

square shape: side length al = 100 $\mu$m, surface area S1 = 10000 $\mu$m$^2$, maximum diagonal length d1max = 141 $\mu$m, regular hexagonal shape: side length al = 107 $\mu$m, surface area S1 = 10000 $\mu$m$^2$, maximum diagonal length d1max = 123.5 $\mu$m

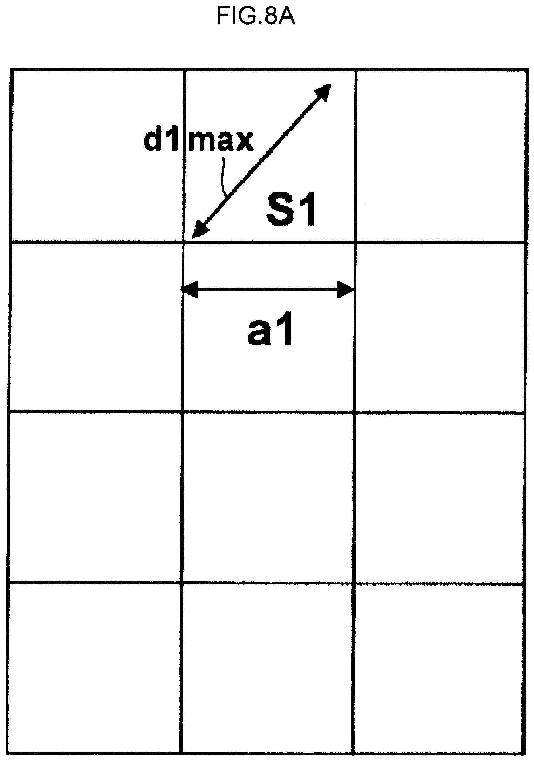

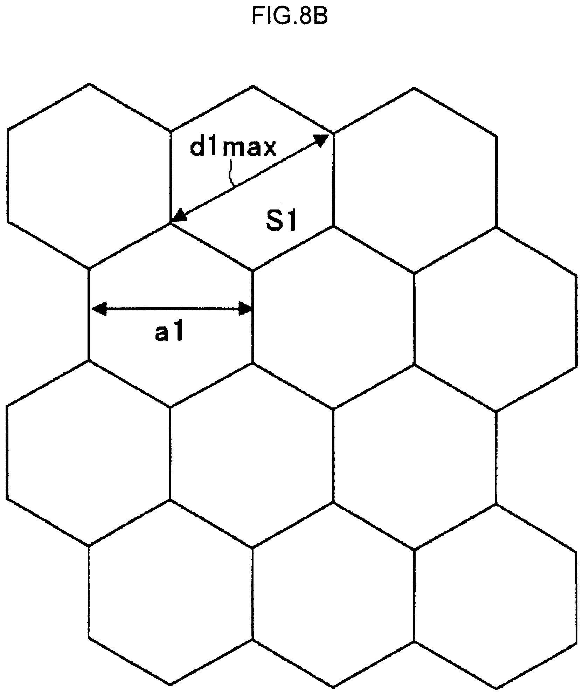

(see also Fig. 8A and Fig. 8B), and so it is possible to reduce the diagonal length d1max by about 12% in comparison to with square shapes for cases of regular hexagonal shapes of the same pixel surface area.

DISCLOSURE OF INVENTION

Technical Problem

In a radiographic image detected employing a detection device using hexagonal shaped pixels as described in for example JP-A No. 2003-255049 and JP-A No. 2006-29839, an image results that has each of the pixels arrayed in a honeycomb pattern. However, most output devices, such as printers and monitors, are configured under the presumption of handling images in which each of the pixels is arrayed in a square lattice pattern. Accordingly, there is a need to perform pixel density conversion by interpolation processing on the detected radiographic image in order to make it conform to such an output device.

However, for example, sometimes pixel data detected by a radiation detection element is wasted when pixel density conversion is performed, depending on the radiation detection element and the definition (resolution) of the finally desired square lattice shaped image. Moreover, for example, in cases in which flattened hexagonal shaped pixels are employed to raise the resolution, if excessive flattening is performed whilst maintaining a high sensitivity (surface area), although the resolution is raised in one direction, there is concern that the resolution will fall in other directions. Namely, depending on the size and the shape of the hexagonal shaped pixels, and depending on the size of the square lattice after pixel density conversion, sometimes this does not lead to both high resolution and sensitivity.

The present invention provides an radiographic imaging device and a radiographic imaging method that may secure high sensitivity and that may increase the resolution.

VAN DE VILLE D ET AL: "Image resampling between orthogonal and hexagonal lattices", International conference on image processing (ICIP), IEEE, vol. 3, 22 September 2002 (2002-09-22), pages 389-392 discloses a method of resampling between orthogonal and hexagonal lattices while loss of information is minimized. Hexagonal splines are constructed and such splines are used to derive the least squares reconstruction function.

Solution to Problem

A first aspect of the present invention is a radiographic imaging device including: a radiation detection element that includes plural same sized hexagonal shaped pixels that detect radiation and are arrayed in a honeycomb pattern; and a pixel density conversion section that performs interpolation processing such that first image data obtained from the radiation detection element is converted into second image data representing an image of plural pixels arrayed in a square grid pattern, wherein, in the radiographic imaging device, the following Formula (1) is satisfied,

$$d2\mathrm{max} \leq d1\mathrm{max} \leq \sqrt{(2 \times S1)} \qquad\qquad \text{Formula (1)}$$

wherein d1max denotes the length of a longest diagonal of the hexagonal shaped pixels, S1 denotes the surface area of the hexagonal shaped pixels, and d2max denotes the length of a diagonal of the square lattice of the second image data.

The condition "d2max $\leq$ d1max" in the front half of the above Formula expresses the condition that the maximum diagonal length of the hexagonal shaped pixels prior to pixel density conversion is the diagonal length of the square lattice after pixel density conversion or greater. By satisfying this condition, the resolution after conversion in all the directions is the resolution prior to conversion or greater, and enables the signals from each of the pixels detected by the radiation detection element to be prevented from being discarded (wasted).

Next, the condition "d1max $\leq \sqrt{(2 \times S1)}$" at the rear half of the Formula expresses the condition that the maximum diagonal length of the hexagonal shaped pixels prior to pixel density conversion is the diagonal length of the square lattice of surface area equivalent to the surface area S1 of the hexagonal shaped pixels, or lower. Namely, d1max represents in a pixel array prior to conversion, an array pitch (maximum pitch) in a direction with the lowest resolution from out of all the directions. However, although hexagonal shaped pixels rather than square shaped pixels are employed in an attempt to achieve higher resolution, when d1max is greater than the diagonal length $\sqrt{(2 \times S1)}$ of a square of surface area S1 the same as that of the hexagonal shaped pixels, the effectiveness of the hexagonal shapes is not sufficiently obtained since the resolution is lowered than by employing square shaped pixels with the same surface area.

Consequently, by configuring so as to satisfy the above Formula, the first aspect of the present invention may achieve both an increase in resolution and sensitivity.

A second aspect of the present invention, in the above first aspect, the hexagonal shaped pixels may be formed in flattened hexagonal shapes.

A third aspect of the present invention, in the above second aspect, the following Formula (2) may also be satisfied.

$$d1\mathrm{max} < \sqrt{((8/3\sqrt{3}) \times S1)} \qquad\qquad \text{Formula (2)}$$

A fourth aspect of the present invention, in the above second aspect or the third aspect, in cases in which the radiographic imaging device is employed as a mammography apparatus to image a breast of an investigation subject, the hexagonal shaped pixels may be formed flattened such that a length in a depth direction from a chest wall side to the tip of the breast is shorter than a width in a direction orthogonal to that direction.

A fifth aspect of the present invention , in the above second aspect to the fourth aspect, the hexagonal shaped pixels may be formed flattened such that one diagonal out of 3 diagonals that pass through the center of the pixels is shorter than the 2 other diagonals and the other 2 diagonals are the same lengths as each other.

A sixth aspect of the present invention, in the above aspects, the pixel density conversion section may perform interpolation processing first in the direction out of a horizontal direction and a vertical direction in the first image data that has a shorter pixel array pitch and then performs interpolation processing in the other direction.

A seventh aspect of the present invention, in the above aspects, may further include: a radiation source that irradiates radiation; and an image output device that outputs an image based on the second image data.

An eighth aspect of the present invention is a radiographic imaging method including: detecting first image data using a radiation detection element that includes plural same sized hexagonal shaped pixels that detect radiation and are arrayed in a honeycomb pattern; and performing interpolation processing such that the first image data is converted into second image data representing an image of plural pixels arrayed in a square grid pattern, wherein in the radiographic imaging method the following Formula (1) is satisfied,

$$d2max \leq d1max \leq \sqrt{(2 \times S1)} \qquad \text{Formula (1)}$$

wherein d1max denotes the length of a longest diagonal of the hexagonal shaped pixels, S1 denotes the surface area of the hexagonal shaped pixels, and d2max denotes the length of a diagonal of the square lattice of the second image data.

A ninth aspect of the present invention, in the above eighth aspect, the following Formula (2) may also be satisfied.

$$d1max < \sqrt{((8/3\sqrt{3}) \times S1)} \qquad \text{Formula (2)}$$

A tenth aspect of the present invention, in the above eighth or the ninth aspect, the hexagonal shaped pixels may be formed in flattened hexagonal shapes.

An eleventh aspect of the present invention, in the above tenth aspect, in cases in which a breast of an investigation subject is imaged, the hexagonal shaped pixels may be formed flattened, and imaging may be performed with the breast disposed such that a shorter pixel width is aligned along a direction from a chest wall side towards the tip of the breast.

A twelfth aspect of the present invention, in the above tenth aspect or the eleventh aspect, wherein imaging may be performed with a radiation detection element in which the hexagonal shaped pixels are formed flattened such that one diagonal out of 3 diagonals that pass through the center of the pixels is shorter than the 2 other diagonals and the other 2 diagonals are the same lengths as each other.

A thirteenth aspect of the present invention, in the above eighth aspect to the twelfth aspect, interpolation processing may be first performed in the direction out of a horizontal direction and a vertical direction in the first image data that has a shorter pixel array pitch and then interpolation processing may be performed in the other direction.

A fourteenth aspect of the present invention, in the above eighth aspect to the thirteenth aspect, may further include outputting an image based on the second image data.

Advantageous Effects of Invention

Consequently, according to the above aspect of the present invention, a radiographic imaging device and radiographic imaging method can be provided with which a high sensitivity can be secured whilst raising the resolution.

BRIEF DESCRIPTION OF DRAWINGS

Fig. 1 is a configuration diagram illustrating a configuration of a configuration of a radiographic imaging system according to an exemplary embodiment.
Fig. 2 is a configuration diagram illustrating an electrical configuration of a radiation detector including a radiation detection element according to an exemplary embodiment.
Fig. 3 is a plan view illustrating a pixel array state of a radiation detection element according to an exemplary embodiment.

Fig. 4 is a cross-section illustrating a structure of a radiation detection element according to an exemplary embodiment.

Fig. 5 is an explanatory diagram for schematically explaining pixel density conversion of a radiographic image detected by a radiation detection element according to an exemplary embodiment.

Fig. 6 are tables illustrating specific examples of check results employing the Formula (1) according to an exemplary embodiment.

Fig. 7 are tables illustrating specific examples of check results employing the Formula (3) according to an exemplary embodiment.

Fig. 8A is a diagram schematically illustrating a specific example of pixel shape and array for detecting radiation.

Fig. 8B is a diagram schematically illustrating a specific example of pixel shape and array for detecting radiation.

Fig. 9 is a cross-section illustrating a structure of a radiation detection element according to an exemplary embodiment.

## BEST MODE FOR CARRYING OUT THE INVENTION

Explanation follows regarding an exemplary embodiment of the present invention, with reference to the drawings. Note that in the following explanation, the present invention is applied to a direct-conversion-type radiation detection element that converts radiation directly into charge.

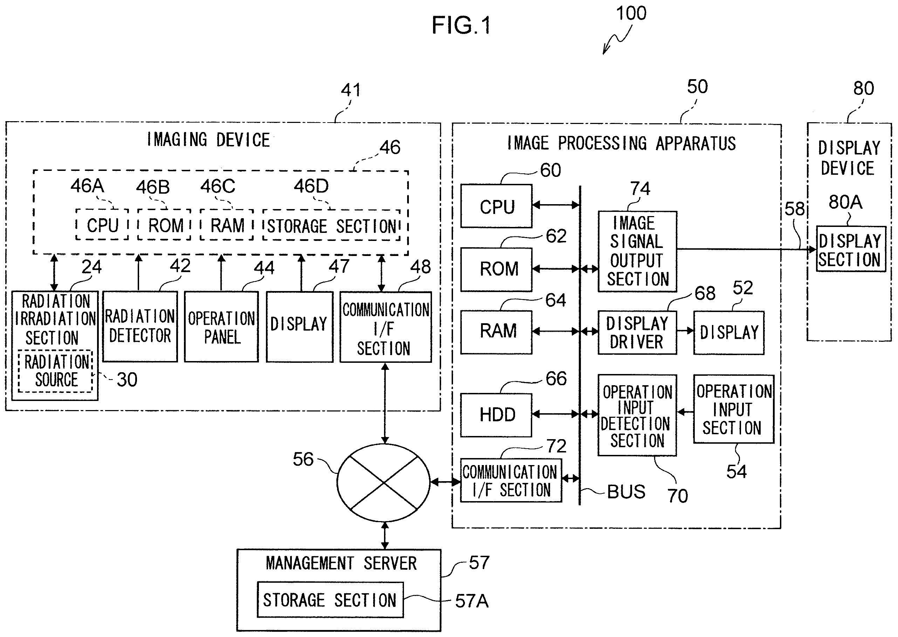

Fig. 1 is a block diagram illustrating a configuration of a radiographic imaging system 100 of the present exemplary embodiment. The radiographic imaging system 100 includes an imaging device 41 that images radiographic images, an image processing apparatus 50, and a display device 80. The image processing apparatus 50 performs image processing on image data expressing imaged radiographic images. The display device 80 displays a subject image expressed by the image data that has been image processed.

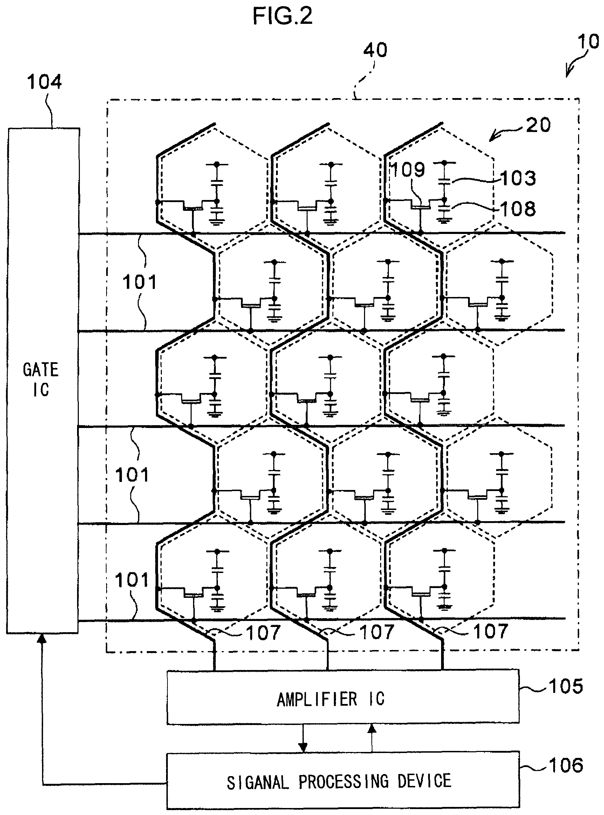

The imaging device 41 includes a radiation detector 42, an operation panel 44, an imaging device control section 46, a display 47 and a communication I/F section 48. The radiation detector 42 includes a radiation detection element 10 (see Fig. 2) that detects a radiographic image. The operation panel 44 is input with imaging conditions, various operation data, and various operation instructions. The imaging device control section 46 controls operation of the apparatus overall. A display 47 displays operation menus and various information. The communication I/F section 48 is connected to a network 56 such as a LAN, and transmits and receives various data to and from other devices connected to the network 56.

The imaging device control section 46 includes a CPU 46A, ROM 46B, RAM 46C, and a non-volatile storage section 46D configured for example by a HDD or flash memory. The imaging device control section 46 is connected to a radiation irradiation section 24, the radiation detector 42, the operation panel 44, the display 47 and the communication I/F section 48. Programs, such as a program for execution by the CPU 46A, are stored in the storage section 46D. Image data, for example, expressing radiographic images is stored in the storage section 46D. For example, when the imaging device 41 of the present exemplary embodiment is employed for mammography, radiographic image data obtained by imaging the breast of an investigation subject is stored in the storage section 46D.

The radiation detector 42 outputs image data expressing a radiographic image to the imaging device control section 46 upon irradiation with radiation. The configuration of the radiation detector 42 will be described in detail later.

The imaging device control section 46 is capable of communicating with the image processing apparatus 50 through the communication I/F section 48 and the network 56, and the imaging device control section 46 performs transmission and reception of various data to and from the image processing apparatus 50.

A management server 57 is also connected to the network 56. The management server 57 is configured including a storage section 57A. The imaging device control section 46 is capable of communicating with the management server 57 through the communication I/F section 48 and the network 56.

The image processing apparatus 50 is configured as a server computer. The image processing apparatus 50 includes a display 52 that displays for example an operation menu and various data, and an operation input section 54 configured including plural keys for inputting various data and operation instructions.

The image processing apparatus 50 further includes a CPU 60, ROM 62, RAM 64, a HDD 66, a display driver 68, an operation input detection section 70, a communication I/F section 72, and an image signal output section 74. The CPU 60 controls operation of the apparatus overall. The ROM 62 is pre-stored with various programs including a control program. The RAM 64 temporarily stores various data. The HDD 66 stores and retains various data. The display driver 68 controls the display of various data on the display 52. The operation input detection section 70 detects operation states with respect to the operation input section 54. The communication I/F section 72 is connected to the imaging device 41 through the network 56, and performs transmission and reception of various data to and from the imaging device 41. The image signal output section 74 outputs image data to the display device 80 through a display cable 58. The image processing apparatus 50 acquires image data expressing radiographic images from the imaging device 41 through the communication IF section 72.

The CPU 60, the ROM 62, the RAM 64, the HDD 66, the display driver 68, the operation input detection section 70, the communication I/F section 72 and the image signal output section 74 are mutually connected together through a system BUS. The CPU 60 is accordingly able to access the ROM 62, the RAM 64 and the HDD 66. The CPU 60 is moreover capable of controlling display of various data on the display 52 through the display driver 68, controlling transmission and reception of various data to and from the imaging device 41 through the communication I/F section 72, and controlling display of images on the display device 80 through the image signal output section 74. The CPU 60 is also capable of ascertaining user operation states to the operation input section 54 through the operation input detection section 70.

Note that, in the image processing apparatus 50, pixel density conversion, described later, is performed on image data expressing a radiographic image detected by the radiation detector 42. A program for performing this pixel density conversion is stored in the ROM 62 or the HDD 66. Image data output to the display device 80 is image data on which pixel density conversion has been performed.

Fig. 2 illustrates an electrical configuration of the radiation detector 42 that employs the radiation detection element 10 according to the present exemplary embodiment.

The radiation detection element 10 is provided with a squared detection region 40 and detects radiation irradiated onto the detection region 40. Plural hexagonal shaped pixels 20 that are the same size as each other are disposed adjacently in the detection region 40. The hexagonal shaped pixels may be regular hexagonal shaped, or may be flattened hexagonal shaped. Note that, included in the definition of the hexagonal shaped pixels 20 are substantially hexagonal shaped pixels wherein each corner portion of the hexagonal shape has been removed. Moreover, the hexagonal shaped pixels 20 also include parallel hexagonal shaped pixels with 3 pairs of parallel facing sides, with all internal angles being 180° or below, rather than a regular hexagonal shape or a flattened substantially hexagonal shape as already described. Moreover, the hexagonal shaped pixels 20 not only include a regular hexagonal shape or a flattened substantially hexagonal shape as already described, but also include parallel hexagonal shaped pixels with 3 pairs of parallel facing sides, with all internal angles being 180° or below. Note that, a regular hexagonal shape means a hexagonal shape in which all 6 sides are exactly equal in length, however in reality there are design limitations and manufacturing tolerances that occur, and so regular hexagonal shape is employed here to include substantially hexagonal shapes that take such factors into consideration.

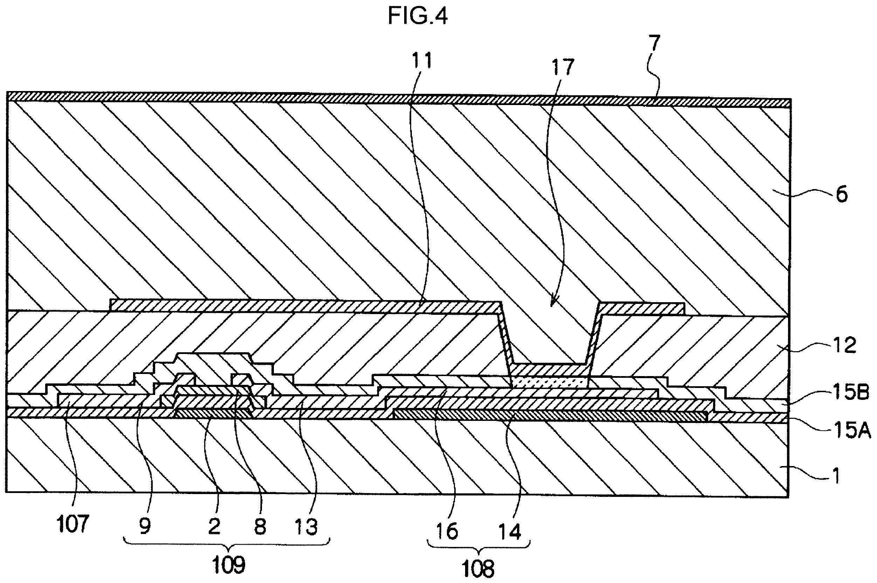

In the present exemplary embodiment, the hexagonal shaped pixels 20 indicate the substantially hexagonal shaped region formed by signal lines 107 illustrated in Fig. 2, however it is sufficient that a honeycomb pattern placement is employed, and there is no limitation to a substantially hexagonal shape. For example, the hexagonal shaped pixels 20 may each be defined as one pixel of a region divided into shapes of the same surface area and the same shape and include a lower portion electrode 11, described later (see Fig. 4), arrayed in a honeycomb pattern (namely tessellated, tiled to fill a surface without gaps). Moreover, the hexagonal shaped pixels 20 may be configured by one pixel of a region divided into shapes of the same surface area and the same profile and include a TFT switch 109, described later (see Fig. 2), in place of the lower portion electrode 11 and are arrayed in a honeycomb pattern. Moreover, the hexagonal shaped pixels 20 may be configured by one pixel of a region divided into shapes of the same surface area and the same profile, and that include not only the lower portion electrode 11 and the TFT switch 109 but also at least one charge storage capacitor 108, and are arrayed in a honeycomb pattern.

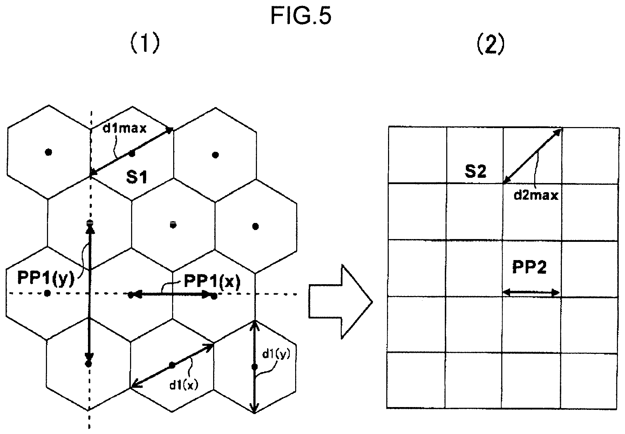

In order to avoid making the pixel density conversion processing more complicated, the shape of the hexagonal shaped pixels 20 is preferably configured such that an axial direction of one diagonal line of the hexagonal shape matches any one axial direction of a square lattice after conversion, and the shapes divided by the axis preferably have symmetry about this axis. Namely, for example as can be seen in Fig. 5, a diagonal line dl(y) of the hexagonal shape matches one axis of the square lattice, and the 2 shapes divided by the diagonal line dl(y) are symmetrical about the diagonal line dl(y).

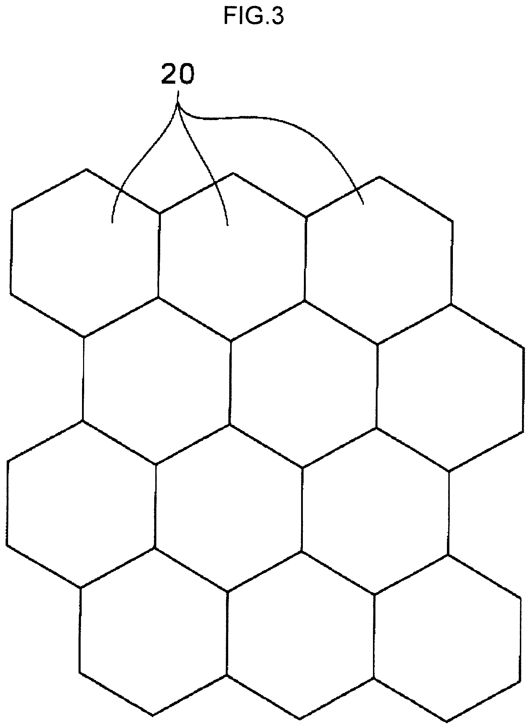

Note that, in the present exemplary embodiment, as illustrated in Fig. 3, each of the pixels 20 is disposed in a honeycomb pattern. In the radiation detection element 10 of the present exemplary embodiment, a first pixel row of plural hexagonal shaped pixels 20 of the same size arrayed along a specific direction, and a second pixel row of plural hexagonal shaped pixels 20 of the same size as the pixels 20 of the first pixel row arrayed along the specific direction, are alternately disposed along a direction intersecting the specific direction. Moreover, in the radiation detection element 10 of the present exemplary embodiment, the pixels 20 of the second pixel row are disposed so as to correspond to the position between adjacent pixels of the first pixel row, such that each of the pixels 20 of the second pixel row are accordingly disposed displaced by 1/2 the array pitch relative to the pixels 20 of the first pixel row. The placement of the honeycomb pattern can be also described as follows when viewed along a column direction. First pixel columns of plural hexagonal shaped pixels 20 of the same size arrayed along a specific direction, and second pixel columns of plural hexagonal shaped pixels 20 of the same size as the pixels 20 of the first pixel column arrayed along the specific direction, are alternately disposed along a direction intersecting the specific direction. Moreover, the pixels 20 of the second pixel column are disposed so as to correspond to between adjacent pixels of the first pixel column, such that each of the pixels 20 of the second pixel column are disposed displaced by 1/2 the array pitch with respect to each of the pixels 20 of the first pixel column.

Note that, when imaging the breast of an investigation subject, configuration may be made such that the hexagonal shaped pixels are formed with a flattened shape, and such that imaging is performed with the short width of the pixels disposed along the direction from the chest wall to the tip of the breast.

Explanation follows regarding a specific example. When configuration is made with flattened hexagonal shaped pixels, in Fig. 2, the pixels are flattened so as to be squashed along the up-down direction of the paper (the same is also true for Fig. 3 and Fig. 5 (1), described later). This is a preferable configuration in a mammography device that performs imaging with the upper portion of the paper in Fig. 2 corresponding to the chest wall. This is, in the case of a mammography device, there is a need to image in fine detail in the depth direction from the chest wall side to the tip of the breast. By making configuration in which the pixels are squashed along in this direction (namely such that the length of the hexagonal shaped pixels 20 in the depth direction from the chest wall side to the breast tip side is shorter than the width in the direction intersecting with the depth direction), and in which external circuits such as a gate IC and an amp IC are not disposed on the chest wall side, imaging in the depth direction from the chest wall side to the breast tip can be performed at fine resolution.

Moreover, when the hexagonal shaped pixels are flattened, configuring such that one diagonal line is shorter than the other 2 diagonal lines, with the other 2 diagonal lines being the same length (referred to as a second flattening method), is preferable, than flattening wherein one diagonal line out of the 3 diagonal lines that cross the center of the pixel is longer than the other 2 diagonal lines, and the other 2 diagonal lines are of the same length (referred to as a first flattening method). Specifically, for example as in the example illustrated in Fig. 5 (1), flattening is preferably configured such that a diagonal length dl(y) in the up-down direction of the paper is shorter than the other 2 diagonal lengths dl(x) (namely squashed along the up-down direction of the paper).

In an un-flattened regular hexagonal shape, in Fig. 5 (1) a horizontal direction pixel array pitch PPI(x) is shorter than a vertical direction pixel array pitch PPI(y). Accordingly, high resolution is secured along the horizontal direction. With high resolution thus secured, flattening with the second flattening method such that the diagonal length dl(y) in the vertical direction is shortened enables high resolution to be secured not only along the horizontal direction but also along the vertical direction. Note that, when the above is employed in the radiation detection element 10, configuration may also be made in which the state illustrated in the drawings is rotated by 90 degrees.

Each of the pixels 20 include a sensor section 103 that receives irradiated radiation and generates charges, a charge storage capacitor 108 that accumulates the charges generated in the sensor section 103, and a TFT switch 109 for reading the charges accumulated in the charge storage capacitor 108.

In the radiation detection element 10, scan lines 101 are disposed respectively one for each pixel row in the horizontal direction of the pixels 20 (referred to below as the scan line direction). The scan lines 101 are connected to the TFT switches 109 that are provided to each of the pixels 20 of pixel rows along the scan line direction, and the scan lines 101 let control signals flow for switching each of the TFT switches 109.

Moreover, in the radiation detection element 10, there are signal lines 107 provided at one line for each of the pixel rows in the vertical direction of the pixels 20 (referred to below as the signal line direction) that snakes so as to circumnavigate each of the pixels 20. The signal lines 107 are connected to each of the TFT switches 109 of the respective pixels 20, and charges that have accumulated in the charge storage capacitors 108 flow according to the switching state of the TFT switches 109.

Plural amplifiers IC 105 for detecting electrical signals flowing out in each of the signal lines 107 are provided at one signal line direction end side of the radiation detection element 10 according to the present exemplary embodiment. A predetermined number of lines of the signal lines 107 are connected to the amplifiers IC 105.

In the radiation detection element 10 according to the present exemplary embodiment, plural gates IC 104 are provided to each of the scan lines 101 at one scan line direction side to output control signals for switching the TFT switches 109 ON/OFF. Each of the scan lines 101 is connected to one of the gates IC 104, with a predetermined number of lines connected thereto. Note that Fig. 1 is simplified and illustrates only one each of the amplifiers IC 105, and the gates IC 104.

Each of the ICs 105 is internally provided with an amplification circuit that amplifies the electrical signal input for each of the signal lines 107. In the amplifiers IC 105, the electrical signals input by each of the signal lines 107 are amplified by the amplification circuit and detected as information for each of the pixels configuring an image, and the charge amounts accumulated in the charge storage capacitors 108 are detected.

Each of the amplifiers IC 105 and the gates IC 104 is connected to a signal processing apparatus 106. The signal processing apparatus 106 performs specific processing on electrical signals detected in the amplifiers IC 105, such as noise reduction or the like. The signal processing apparatus 106 outputs a control signal to the amplifiers IC 105 representing a timing for signal detection, and outputs a control signal to the gates IC 104 representing a timing to output a scan signal.

Fig. 4 is a cross-section illustrating a structure of the radiation detection element 10.

As illustrated in Fig 4, the radiation detection element 10 is formed on an insulating substrate 1 with the scan lines 101, storage capacitor lower electrodes 14, and gate electrodes 2. One of the scan lines 101 is formed for each of the pixel rows of the pixels 20 in the scan line direction, by snaking so as to circumnavigate each of the pixels 20 between each of the pixel rows. The scan lines 101 are connected to the gate electrodes 2 formed to each of the pixels 20 at the upper side of the pixel rows, and are connected to the storage capacitor lower electrodes 14 formed to each of the pixels 20 at the lower side of the pixel rows. The wiring layer in which the scan lines 101, the storage capacitor lower electrodes 14 and the gate electrodes 2 are formed (this wiring layer is referred to below as the "first signal wiring layer") is formed of Al or Cu, or using stacked layers formed mainly from Al or Cu. However, the material of the first signal wiring layer is not limited thereto.

An insulating film 15A is formed on one face above the first signal wiring layer, with locations positioned above the gate electrodes 2 acting as gate insulating films in the TFT switches 109. The insulating film 15A is configured, for example, from $SiN_x$ by, for example, film forming using Chemical Vapor Deposition (CVD).

Semiconductor active layers 8 are formed with island shapes on the insulation film 15A above each of the gate electrodes 2. The semiconductor active layers 8 are channel portions of the TFT switches 109 and are, for example, formed from an amorphous silicon film.

Source electrodes 9 and drain electrodes 13 are formed in a layer above. The signal lines 107 are formed in the wiring layers formed with the source electrodes 9 and the drain electrodes 13, and storage capacitor upper electrodes 16 are formed above the insulating film 15A at positions corresponding to the storage capacitor lower electrodes 14. The drain electrodes 13 are connected to the storage capacitor upper electrodes 16. The signal lines 107 are connected to the source electrodes 9 formed to each of the pixels 20. The wiring layer in which the source electrodes 9, the drain electrodes 13, the signal lines 107, and the storage capacitor upper electrodes 16 are formed (this wiring layer is referred to below as the "second signal wiring layer") is formed of Al or Cu, or using stacked layers formed mainly from Al or Cu. However, the material of the second signal wiring layer is not limited thereto. An impurity doped semiconductor layer (not illustrated in the drawings) is formed between the source electrodes 9 and the drain electrodes 13, and the semiconductor active layers 8 from impurity doped amorphous silicon or the like. The TFT switches 109 used in switching are accordingly configured therefrom. Note that in the TFT switches 109, the source electrodes 9 and the drain electrodes 13 may be reversed, depending on the polarity of the charges that are collected and accumulated in lower electrodes 11, described later.

In cases, such as described above, in which 1 pixel of hexagonal shaped pixels 20 is defined by a region divided in a honeycomb shape including lower electrodes 11 of the same surface area and same shape, the lower electrodes 11 are disposed within the shape of the hexagonal shaped pixels 20. Consequently, the shape of the lower electrodes 11 is preferably formed with the same hexagonal shape as the hexagonal shaped pixels 20 so as to enable the signal charges generated by electromagnetic radiation to be collected with better efficiency, arrayed in a honeycomb pattern. However, the shape of the lower electrodes 11 is not limited to the above, and any shape may be employed therefor as long as it is contained within the shape of the hexagonal shaped pixels 20, such as being formed in a substantially hexagonal shape with corners removed, a quadrilateral shape, a circular shape or an octagonal shape.

A TFT protection film layer 15B is formed so as to cover the second signal wiring layer, over substantially the entire surface of the region (substantially the entire region) provided with pixels above the substrate 1. The TFT protection film layer 15B is, for example, configured from $SiN_x$ or the like, by for example CVD film forming.

A coated interlayer insulating film 12 is formed on the TFT protection film layer 15B. The interlayer insulating film 12 is formed from a low permittivity (specific permittivity $\varepsilon_r$ = 2 to 4) photosensitive organic material (examples of such materials include positive working photosensitive acrylic resin materials with a base polymer formed by copolymerizing methacrylic acid and glycidyl methacrylate, mixed with a naphthoquinone diazide positive working photosensitive agent) at a film thickness of 1 $\mu$m to 4 $\mu$m.

In the radiation detection element 10 according to the present exemplary embodiment, the capacitance between metal disposed in the layers above the interlayer insulating film 12 and below the interlayer insulating film 12 is suppressed small by the interlayer insulating film 12. Generally such materials also function as a flattening film, exhibiting an effect of flattening out steps in the layers below. In the radiation detection element 10 according to the present exemplary embodiment, contact holes 17 are formed in the interlayer insulating film 12 and the TFT protection layer 15B at positions corresponding to the storage capacitor upper electrodes 16.

The lower electrodes 11 of each of the sensor portions 103 are formed on the interlayer insulating film 12 for each of the pixels 20, so as to cover the pixel region while also filling each of the contact holes 17. The lower electrodes 11 are formed from an amorphous transparent conducting oxide film (ITO) and are connected to the storage capacitor upper electrodes 16 through the contact holes 17. As a result, the lower electrodes 11 and the TFT switches 109 are electrically connected through the storage capacitor upper electrodes 16.

A semiconductor layer 6 is uniformly formed on the lower electrodes 11 over substantially the entire surface of the pixel region where the pixels 20 are provided on the substrate 1. The semiconductor layer 6 generates charges (electrons-holes) internally on irradiation with radiation such as X-rays. In other words, the semiconductor layer 6 has electrical conduction properties and is employed to convert image data from X-rays into charge data. For example, the semiconductor layer 6 may be formed from amorphous selenium (a-Se) having selenium as the main component. Note that reference to the main component means contained at a ratio of 50% of more.

An upper electrode 7 is formed on the photoelectric conversion layer 6. The upper electrode 7 is connected to a bias power source (not illustrated in the drawings) and supplies a bias voltage from the bias power source.

Explanation next follows regarding operating principles of the radiation detector 42 according to the present exemplary embodiment.

Charges (electron-hole pairs) are generated in the semiconductor layer 6 when X-rays are irradiated onto the semiconductor layer 6 in a state in which a bias voltage is being applied between the upper electrode 7 and the storage capacitor lower electrodes 14.

The semiconductor layer 6 and the charge storage capacitors 108 are structured so as to be electrically connected in series. Electrons generated in the semiconductor layer 6 accordingly migrate to the + (plus) electrode side and holes migrate to the - (minus) electrode side. During image detection, an OFF signal (0V) is output from the gates IC 104 to all of the scan lines 101, and a negative bias voltage is applied to the gate electrodes 2 of the TFT switches 109. Each of the TFT switches 109 is accordingly maintained in an OFF state. As a result, the electrons generated in the semiconductor layer 6 are collected by the lower electrodes 11 and accumulated in the charge storage capacitors 108.

During image reading an ON signal is output one line at a time in sequence to each of the scan lines 101, and an ON signal (+ 10 to 20V) is applied through the scan lines 101 to the gate electrodes of the TFT switches 109. The TFT switches 109 of each of the pixels 20 are thereby switched on one row at a time through each of the pixel rows in the scan line direction, and electrical signals according to the charge amounts accumulated in the charge storage capacitors 108 of each of the pixels 20 flow out to the signal lines 107 one line at a time.

The amplifiers IC 105 detect the charge amounts accumulated in the charge storage capacitors 108 of each of the sensor section 103 based on the electrical signals flowing in each of the signal lines 107, as data of pixels configuring an image (referred to below as pixel data). This thereby enables image data to be obtained representing an image expressing X-rays irradiated onto the radiation detection element 10.

Image data obtained by the radiation detection element 10 according to the present exemplary embodiment is image data expressing an image configured by pixels that are each arrayed in a honeycomb pattern. However, most output devices such as printers and monitors (the display device 80 in the present exemplary embodiment) are configured under the presumption to handle images with pixels that are each arrayed in a square lattice pattern. Thus in the present exemplary embodiment, interpolation processing is performed by the image processing apparatus 50 on the image data representing a detected radiographic image so as to perform pixel density conversion.

Fig. 5 is a schematic diagram that schematically illustrates contents of pixel density conversion processing.

As explained with reference to Fig. 2 and Fig. 3, each of the hexagonal shaped pixels is arrayed in a honeycomb pattern, hence a radiographic image detected by the radiation detection element 10 is, as illustrated in Fig. 5 (1) an image with each of the pixels arrayed in a honeycomb pattern. Note that the black dots illustrated within the pixels are the centers of gravity of the pixels.

The image data representing this radiographic image is, as illustrated in Fig. 5 (2), converted into image data representing an image in which each of the plural pixels are arrayed in a square lattice pattern. Note that, when this is performed, the surface area S2 of the square lattice of the image after conversion is converted so as to be the surface area S1 of the hexagonal shaped pixels prior to conversion. Known interpolation processing such as a nearest neighbor method, a linear interpolation method, or a bi-cubic interpolation method may be employed for interpolation processing performed in pixel density conversion. Moreover, for example, configuration may be made such that the pixel density conversion method described in JP-A No. 2000-244733 is performed.

Hereinbelow, image data representing an image with each of the pixels arrayed in a honeycomb pattern prior to pixel density conversion is called first image data, and image data representing an image with each of plural pixels arrayed in a square lattice pattern after pixel density conversion is called second image data.

In such pixel density conversion, it is preferable for the hexagonal shaped pixels configuring the radiation detection element 10 to be formed such that the high resolution obtained by the radiation detection element 10 across all directions is not wasted (such that each of the detected signals is not wasted), or such that there is no drop in the resolution while high sensitivity is maintained.

Thus in the present exemplary embodiment, the hexagonal shaped pixels are formed such that the following relationship of Equation (1) is satisfied between the hexagonal shaped pixels configuring the radiation detection element 10 and the square lattice after pixel density conversion. Note that the size of the square lattice may also be adjusted when performing pixel density conversion such that Formula (1) is satisfied.

$$\mathrm{d2max} \leq \mathrm{d1max} \leq \sqrt{(2 \times \mathrm{S1})} \qquad\qquad \text{Formula (1)}$$

Here, d2max denotes the length (diagonal length) of a diagonal of the square lattice of the second image data after pixel density conversion; d1max denotes the length of the longest diagonal out of the diagonals of the hexagonal shaped pixels prior to pixel density conversion (also referred to below as the maximum diagonal length); and S1 denotes the surface area of the hexagonal shaped pixels.

Detailed explanation follows regarding the above Formula (1).

First, the condition "d2max $\leq$ d1max" in the front half of Formula (1) expresses that the maximum diagonal length of the hexagonal shaped pixels prior to pixel density conversion is the diagonal length of the square lattice after pixel density conversion or greater. Satisfying this condition gives a resolution in all the directions after conversion is equal to or greater than the resolution prior to conversion, and enables the signals from each of the pixels detected by the radiation detection element 10 to be prevented from being discarded (wasted).

Next, the condition "d1max $\leq \sqrt{(2 \times S1)}$" at the rear half of Formula (1) expresses the condition that the maximum diagonal length of the hexagonal shaped pixels prior to pixel density conversion is the diagonal length of the square lattice of surface area equivalent to the surface area S1 of the hexagonal shaped pixels, or lower. In the following, $\sqrt{(2 \times S1)}$ is also referred to as d0max.

d1max here in a pixel array prior to conventional represents an array pitch (maximum pitch) in a direction with the lowest resolution from out of all the directions. Fundamentally a higher resolution is achieved by employing hexagonal shaped pixels instead of lattice shaped pixels. However, when d1max is greater than the diagonal length d0max of a square of surface area S1 the same as that of the hexagonal shaped pixels, the effectiveness of the hexagonal shapes is not sufficiently obtained, since the resolution is lower than employing square shaped pixels with the same surface area.

Consequently, excellent sensitivity can be exhibited by forming hexagonal shaped pixels so as to satisfy the condition in the rear half, and the resolution can also be raised. For example, even in cases in which the hexagonal shapes are flattened and the resolution raised, a high resolution can be secured in all the directions by forming with a flattening ratio such that these conditions are satisfied for each of the pixels of the radiation detection element 10.

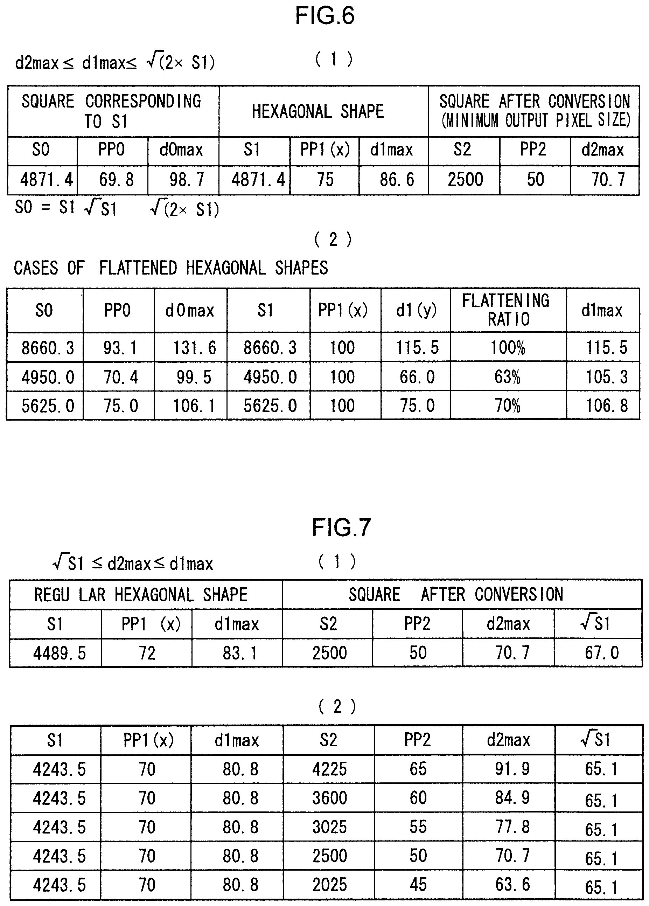

Specific examples are now given, examining whether or not Formula (1) is satisfied with reference to Fig. 6.

First, at Fig. 6 (1) the shape of the hexagonal shaped pixels prior to pixel density conversion is prescribed as follows (see also Fig. 5 (1)).

surface area S1 = 4871.1$\mu$m$^2$
horizontal direction pixel array pitch PP1 (x) = 75$\mu$m
maximum diagonal length d1max = 86.6$\mu$m

The square shape with equivalent surface area to the surface area S1 of the hexagonal shaped pixels is expressed with the following numerical values.

surface area S0 = S1 = 4871.4$\mu$m$^2$
side length PP0 = $\sqrt{(S1)}$ = 69.8$\mu$m
diagonal length d0max = $\sqrt{(2 \times S1)}$ = 98.7$\mu$m

In cases in which the array pitch (= the side length of the square lattice) pp2 of the pixels after pixel density conversion is 50$\mu$m, the surface area S2 of the pixels and the diagonal length d2max is the following (see also Fig. 5 (2)).

surface area S2 = 2500$\mu$m$^2$
diagonal length d2max = 70.7$\mu$m

In such cases, since d1max = 86.6$\mu$m and d2max = 70.7$\mu$m, the front half portion "d2max $\leq$ d1max" of Formula (1) is satisfied.

Moreover, since d1max = 86.6$\mu$m and d0max = 98.7$\mu$m, the condition "d1max $\leq$ d0max" in the rear half portion of Formula (1) is also satisfied.

Explanation next follows regarding an example of flattened hexagonal shaped pixels. Here the flattening is made under the condition such that one diagonal of the diagonals passing through the center of the pixels is made shorter than the other 2 diagonal, and the lengths of the other 2 diagonals are the same as each other. Explanation follows regarding specific examples of a relationship between the Formula (1) and the hexagonal shaped pixel flattening ratio, with reference to Fig. 6 (2). Explanation focusses on the condition in the rear half of Formula (1).

In the table of Fig. 6 (2), the d1(y) is a length of one diagonal out of the diagonals passing through the center of the hexagonal shaped pixels (this being the vertical direction diagonal length in Fig. 5 (1)). When the length of the remaining two diagonals (with equal lengths to each other) out of the diagonals passing through the center of the hexagonal shaped pixels excluding the vertical direction diagonal is denoted d1(x), the value expressed by d1(y)/d1(x) is the flattening ratio. In cases in which flattening of the pixels is performed so as to squash in the vertical direction using the above second flattening method, d1(x) = d1max.

As illustrated in Fig. 6 (2), the condition "d1max $\leq$ d0max" of the rear half of Formula (1) is satisfied in cases in which the flattening ratio is 100%. d1max is substantially equal to d0max when the flattening ratio is 70%. In cases in which the flattening ratio is 63%, the condition of the rear half of Formula (1) is no longer satisfied due to d1max greatly exceeding the d0max. This illustrates that the flattening ratio needs to be about 70% or greater in order to satisfy the condition of the rear half of Formula (1). A flattening ratio is defined by Formula (1) so as to exhibit high resolution and excellent sensitivity.

Preferably, the hexagonal shaped pixels are flattened so as to satisfy the following Formula (2). The resolution is thereby raised higher than for regular hexagonal shaped pixels.

$$d1max < \sqrt{(8/3\sqrt{3})} \times S1 \qquad\qquad \text{Formula (2)}$$

Moreover, in order that the relationship between the hexagonal shaped pixels configuring the radiation detection element 10 and the square lattice after pixel density conversion satisfies the following Formula (3), either the size of the square lattice after pixel density conversion is adjusted, or the size of the square lattice after pixel density conversion is adjusted and the hexagonal shaped pixels formed such that the following Formula (3) is satisfied.

$$\sqrt{S1} \leq d2max \leq d1max \qquad\qquad \text{Formula (3)}$$

Details explanation follows regarding Formula (3).

First, for the condition "d2max $\leq$ d1max" of the rear half of Formula (3), similarly to the rear half portion of Formula (1), satisfying this condition gives a resolution in all the directions after conversion that is the resolution prior to conversion or greater, and enables the signals from each of the pixels detected by the radiation detection element 10 to be prevented from being discarded (wasted).

Next, the condition "$\sqrt{S1} \leq$ d2max" at the front half of Formula (3) expresses that the diagonal length of the square lattice after pixel density conversion is equal to or greater than the side length of the square lattice of surface area equivalent to the surface area S1 of the hexagonal shaped pixels prior to pixel density conversion (also the array pitch of the pixels in the horizontal direction and the vertical direction). In the following $\sqrt{(S1)}$ is called PP0.

Here, d2max in the pixel array after conversion indicates that the array pitch (maximum pitch) in the direction with the lowest resolution out of all the directions. PP0 indicates the array pitch (maximum pitch) in the radiation detection element in the direction with the highest resolution in the square lattice with the same surface area as the hexagonal shaped pixels prior to conversion. In the present exemplary embodiment, although normally it is desired to detect with square lattice pixels, a radiographic image is detected by employing hexagonal shaped pixels, and performs pixel density conversion, instead of using square shaped pixels, so as to obtain sufficient sensitivity with the finally desired resolution. Consequently, the resolution after pixel density conversion does not need to be as high as the resolution of the originally desired square lattice. Namely, the maximum pitch d2max of the square lattice after pixel density conversion does not need to be smaller than the minimum pitch PP0 of the square lattice of the same surface area as the surface area S1 of hexagonal shaped pixels employed to obtain the originally desired resolution. If d2max is smaller than the PP0, a wasteful increases in the size of the second image data after conversion, leading to a corresponding drop in the processing speed.

Consequently, satisfying the condition of the rear half of Formula (3) prevents the size of the second image data after conversion from becoming too large, and prevents a drop in processing speed.

A specific example is given with reference to Fig. 7 to examine whether or not Formula (3) is satisfied.

First, in the example illustrated in Fig. 7 (1), the shape of the regular hexagonal shaped prior to pixel density conversion is defined as follows (see also Fig. 5 (1)).

surface area S1 = 4489.5$\mu$m$^2$
horizontal direction pixel array pitch PP1 (x) = 72$\mu$m
maximum diagonal length d1max = 83.1$\mu$m

A square shape of equivalent surface area to that of the surface area S1 of regular hexagonal shaped pixels is expressed numerically as follows.

surface area S0 = S1 = 4489.5$\mu$m$^2$
side length PP0 = $\sqrt{(S1)}$ = 67.0$\mu$m

In cases in which the array pitch (= the side length of the square lattice) pp2 of the pixels after pixel density conversion is $50\mu$m, the surface area S2 of the pixels and the diagonal length d2max is the following (see also Fig. 5 (2)).

surface area S2 = $2500\mu$m$^2$
diagonal length d2max = $70.7\mu$m

In such cases, since d2max = $70.7\mu$m and PP0 = $\sqrt{(S1)}$ = $67.0$ $\mu$m, the front half portion "$\sqrt{(S1)} \leq$ d2max" of Formula (3) is satisfied.

Moreover, since d1max = $83.1\mu$m and d2max = $70.7\mu$m, the condition "d2max < d1max" in the rear half portion of Formula (3) is also satisfied.

Next, the value of PP2 is changed in a case in which the maximum diagonal length of the regular hexagonal shaped pixels d1max = $80.8\mu$m, and the size of an appropriate square lattice after pixel density conversion is derived.

As illustrated in Fig. 7 (2), PP2 is changed from $65\mu$m to $45\mu$m at intervals of $5\mu$m, and examination made into whether or not Formula (3) is satisfied. As illustrated in Fig. 7 (2), PP2 is $65\mu$m, $60\mu$m, $55\mu$m and $50\mu$m in cases in which the condition "$\sqrt{(S1)}$ < d2max" of the front half portion of Formula (3) is satisfied, however the front half portion condition is not satisfied in a case in which PP2 is $45\mu$m.

The condition "d2max $\leq$ d1max" of the rear half portion of Formula (3) is satisfied in cases in which PP2 is $55\mu$m, $50\mu$m and $45\mu$m, however the rear half portion condition is not satisfied in cases in which PP2 is $65\mu$m and $60\mu$m.

Consequently, cases in which both the front half portion and the rear half portion of Formula (3) are satisfied are when PP2 is $55\mu$m and $50\mu$m. Thus, in cases in which regular hexagonal shaped pixels with a PP1(x) of $70\mu$m arrayed in a honeycomb pattern are employed in a radiation detection element for detecting a radiographic image, the array pitch (side length) PP2 of square shaped pixels after pixel density conversion may be $55\mu$m or $50\mu$m.

However, configuration may be made in which, in the interpolation processing in pixel density conversion, interpolation processing may be performed in a direction with short array pitch of pixels in first image data out of the horizontal direction and the vertical direction, before performing interpolation processing in the other direction. Performing the interpolation processing from the shorter separation direction gives higher precision than in the other direction, and also raises the conversion speed.

For example, in cases in which the horizontal direction pixel array pitch PP1 (x) is shorter than the vertical direction pixel array pitch PP1 (y), the interpolation processing is performed first in the horizontal direction, and then the interpolation processing in the vertical direction is performed after the horizontal direction processing. Moreover, in cases in which the vertical direction pixel array pitch PP1 (y) is shorter than the horizontal direction pixel array pitch PP1 (x), the interpolation processing in the vertical direction is performed first, and then the horizontal direction interpolation processing is performed after the horizontal direction interpolation processing.

Thus the radiation detection element 10 of the present exemplary embodiment enables detected data to be utilized sufficiently.

Note that in the above exemplary embodiment, the array state of the gates IC 104 and the amplifiers IC 105, the connection state of the signal lines 107, and the connection state of the scan lines 101 are merely examples thereof, and there is no restriction to the above exemplary embodiments.

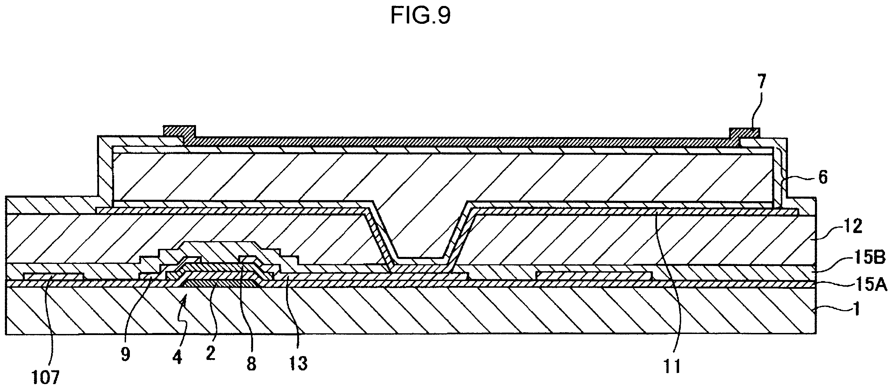

Moreover, in the exemplary embodiments, explanation has been given of a case in which application is to a direct-conversion-type radiation detection element 10, however application may be made to an indirect-conversion-type radiation detection element 10. Fig. 9 illustrates an example of an indirect-conversion-type radiation detection element 10. In an indirect conversion method a scintillator layer (not illustrated in the drawings, such as CsI:T1, GOS ($Gd_2O_2S$:Tb) or the like) that first converts radiation into light is provided in the radiation detection element 10 to the detection region 40. In an indirect-conversion-type radiation detection element 10, similarly to the direct-conversion-type semiconductor layer 6 and the lower electrodes 11 illustrated in Fig. 4, a semiconductor layer 6 for converting converted light into charges and lower electrodes 11 for collecting the charges are provided in each of the pixels below the scintillator layer. Moreover, in an indirect-conversion-type radiation detection element 10, switches 4 are provided for reading charges that have been collected by the lower electrodes 11. Other parts of the configuration of the indirect-conversion-type radiation detection element 10 may appropriately include the direct conversion configuration illustrated in Figs. 1, 2, 3, 4.

In an indirect conversion case, the semiconductor layer 6 that converts light into charge may be partitioned into each of the pixels 20, or the upper electrode 7 that supplies a bias to the semiconductor layer 6 may be partitioned into each of the pixels 20. In cases in which the above exemplary embodiment is applied to the indirect-conversion-type radiation detection element 10, the pixels 20 are each determined as single pixels of partitioned hexagonal shape regions containing at least one of the lower electrode 11, the semiconductor layer 6, the upper electrode 7 or the switching element 4 shaped with the same surface area and same shape as each other and arrayed in a honeycomb pattern (namely tessellated, tiled to fill a surface without gaps). However, the definition of the pixels 20 is not limited thereto, and substantially hexagonal shaped regions formed by the signal lines 107 may be defined as the single pixels.

In the exemplary embodiment, explanation has been given of an example in which the functionality of a pixel density conversion section that performs pixel density conversion is imparted to the image processing apparatus 50, and the image processing apparatus 50 is configured as a separate device to the imaging device 41 containing the radiation detection element 10. However the present invention is not limited thereto. For example, configuration may be made such that, out of an image density conversion section or the image processing apparatus 50, the section having an image processing function to perform pixel density conversion is provided in the radiation detector 42. Moreover, as a separate example, a system may be configured with the imaging device 41 and the image processing apparatus 50 that performs pixel density conversion connected together through a network.

Moreover, in the above exemplary embodiment, explanation has been given of a case in which the present invention is applied to the radiation detector 42 that detects an image by detecting X-rays as the detection target radiation. However the present invention is not limited thereto. For example, any of visible light, ultraviolet light, infrared radiation, alpha particles, gamma rays or the like may be employed as the detection target radiation.

Moreover, in the present exemplary embodiment, explanation has been given of a case in which in the radiation detection element 10 the pixels 20 are each equipped with the charge storage capacitors 108. However, sometimes each of the pixels 20 may be configured without being formed with the charge storage capacitors 108, such as in cases in which the lower electrodes 11 already have sufficient capacity to store charges.

The configuration of the radiographic imaging system 100 and the configuration of the radiation detection element 10 explained in the above exemplary embodiment are also merely examples thereof, and it is possible to make appropriate modifications thereto within a range not departing from the spirit of the present invention.

**Claims**

1. A radiographic imaging device comprising:

   a radiation detection element including a plurality of same sized hexagonal shaped pixels that detect radiation and are arrayed in a honeycomb pattern; and
   a pixel density conversion section that performs interpolation processing such that first image data obtained from the radiation detection element is converted into second image data representing an image of a plurality of pixels arrayed in a square grid pattern,
   wherein the pixel conversion is so performed that the following Formula (1) is satisfied in the radiographic imaging device,

   $$\text{d2max} \leq \text{d1max} \leq \sqrt{(2 \times \text{S1})} \qquad\qquad \text{Formula (1)}$$

   wherein d1max denotes the length of a longest diagonal of the hexagonal shaped pixels, S1 denotes the surface area of the hexagonal shaped pixels, and d2max denotes the length of a diagonal of the square lattice of the second image data.

2. The radiographic imaging device of claim 1, wherein the hexagonal shaped pixels are formed in flattened hexagonal shapes.

3. The radiographic imaging device of claim 2, wherein, in cases in which the radiographic imaging device is employed as a mammography apparatus to image a breast of an investigation subject, the hexagonal shaped pixels are formed flattened such that a length in a depth direction from a chest wall side to the tip of the breast is shorter than a width in a direction orthogonal to that direction.

4. The radiographic imaging device of claim 2 or claim 3, wherein the hexagonal shaped pixels are formed flattened such that one diagonal, out of 3 diagonals that pass through the center of the pixels, is shorter than the 2 other diagonals, and the other 2 diagonals are the same lengths as each other.

5. The radiographic imaging device of any one of claim 1 to claim 4, wherein the pixel density conversion section performs interpolation processing first in the direction out of a horizontal direction and a vertical direction in the first image data that has a shorter pixel array pitch and then performs interpolation processing in the other direction.

6. The radiographic imaging device of any one of claim 1 to claim 5, further comprising:

a radiation source that irradiates radiation; and
an image output device that outputs an image based on the second image data.

**7.** A radiographic imaging method comprising:

detecting first image data using a radiation detection element that includes a plurality of same sized hexagonal shaped pixels that detect radiation and are arrayed in a honeycomb pattern; and
performing interpolation processing such that the first image data is converted into second image data representing an image of a plurality of pixels arrayed in a square grid pattern,
wherein, in the radiographic imaging method, the hexagonal pixels and the pixels in the square grid are so configured that the following Formula (1) is satisfied,

$$d2max \leq d1max \leq \sqrt{(2 \times S1)} \qquad \text{Formula (1)}$$

wherein d1max denotes the length of a longest diagonal of the hexagonal shaped pixels, S1 denotes the surface area of the hexagonal shaped pixels, and d2max denotes the length of a diagonal of the square lattice of the second image data.

**8.** The radiographic imaging method of claim 7, wherein the hexagonal shaped pixels are formed in flattened hexagonal shapes.

**9.** The radiographic imaging method of claim 8, wherein, in cases in which a breast of an investigation subject is imaged, the hexagonal shaped pixels are formed flattened, and imaging is performed with the breast disposed such that a shorter pixel width is aligned along a direction from a chest wall side towards the tip of the breast.

**10.** The radiographic imaging method of any one of claim 8 to claim 9, wherein imaging is performed with a radiation detection element in which the hexagonal shaped pixels are formed flattened such that one diagonal, out of 3 diagonals that pass through the center of the pixels, is shorter than the 2 other diagonals, and the other 2 diagonals are the same lengths as each other.

**11.** The radiographic imaging method of any one of claim 7 to claim 10, wherein the interpolation processing is first performed in the direction out of a horizontal direction and a vertical direction in the first image data that has a shorter pixel array pitch and then interpolation processing is performed in the other direction.

**12.** The radiographic imaging method of any one of claim 7 to claim 11, further comprising outputting an image based on the second image data.

**Patentansprüche**

**1.** Radiographische Bildgebungsvorrichtung, umfassend:

ein Strahlungsdetektorelement mit mehreren gleich großen hexagonalförmigen Pixeln, die Strahlung detektieren und in einem Honigwabenmuster angeordnet sind; und
einen Pixeldichte-Wandlerabschnitt, der eine Interpolationsverarbeitung in der Weise ausführt, dass erste von dem Strahlungsdetektorelement gewonnene Bilddaten umgewandelt werden in zweite Bilddaten, die ein Bild aus mehreren in einem quadratischen Gittermuster angeordneten Pixeln repräsentieren,
wobei die Pixelumwandlung derart ausgeführt wird, dass folgende Formel (1) in der radiographischen Bildgebungsvorrichtung erfüllt ist:

$$d2max \leq d1max \leq \sqrt{(2 \times S1)} \qquad \text{Formel (1)}$$

wobei d1max die Länge einer längsten Diagonalen der hexagonalförmigen Pixel bezeichnet, S1 die Flächengröße der hexagonalförmigen Pixel bezeichnet und d2max die Länge einer Diagonalen des quadratischen Gitters der zweiten Bilddaten bezeichnet.

**2.** Vorrichtung nach Anspruch 1, bei der die hexagonalförmigen Pixel zu abgeflachten Hexagonalformen geformt sind.

**3.** Vorrichtung nach Anspruch 2, bei der in Fällen, in denen die radiographische Bildgebungsvorrichtung als Mammographievorrichtung zum Abbilden einer Brust einer untersuchten Person verwendet wird, die hexagonalförmigen Pixel derart abgeflacht gebildet sind, dass eine Länge in einer Tiefenrichtung von einer Brustwandseite zu der Spitze der Brust kürzer ist als eine Breite in einer dazu orthogonalen Richtung.

**4.** Vorrichtung nach Anspruch 2 oder 3, bei der die hexagonalförmigen Pixel derart abgeflacht gebildet sind, dass eine von drei durch das Zentrum der Pixel laufenden Diagonalen kürzer ist als die zwei anderen Diagonalen, und die zwei anderen Diagonalen gleiche Länge aufweisen.

**5.** Vorrichtung nach einem der Ansprüche 1 bis 4, bei der der Pixeldichte-Wand-lerabschnitt eine Interpolationsverarbeitung zuerst in der einen Richtung von einer horizontalen Richtung und einer vertikalen Richtung unter den ersten Bilddaten, die einen kürzeren Pixelanordnungs-Mittenabstand aufweisen, ausführt und anschließend die Interpolationsverarbeitung in einer anderen Richtung ausführt.

**6.** Vorrichtung nach einem derAnsprüche 1 bis 5, weiterhin umfassend:

eine Strahlungsquelle, die Strahlung abgibt; und
eine Bildausgabeeinrichtung, die ein Bild basierend auf den zweiten Bilddaten ausgibt.

**7.** Radiographisches Bildgebungsverfahren, umfassend:

Detektieren erster Bilddaten unter Verwendung eines Strahlungsdetektorelements, welches mehrere gleich große hexagonalförmige Pixel enthält, die Strahlung detektieren und in einem Honigwabenmuster angeordnet sind; und
Ausführen einer Interpolationsverarbeitung derart, dass die ersten Bilddaten umgewandelt werden in zweite Bilddaten, die ein Bild mehrerer Pixel repräsentieren, die in einem quadratischen Gittermuster angeordnet sind, wobei in dem radiographischen Bildgebungsverfahren die hexagonalen Pixel und die Pixeln in dem quadratischen Gitter derart konfiguriert sind, dass die folgende Formel (1) erfüllt ist:

$$d2max \leq d1max \leq \sqrt{(2 \times S1)} \qquad \text{Formel (1)}$$

wobei d1max die Länge einer längsten Diagonalen der hexagonalförmigen Pixel bezeichnet, S1 die Flächengröße der hexagonalförmigen Pixel bezeichnet und d2max die Länge einer Diagonalen des quadratischen Gitters der zweiten Bilddaten bezeichnet.

**8.** Verfahren nach Anspruch 7, bei dem die hexagonalförmigen Pixel abgeflachte hexagonale Formen aufweisen.

**9.** Verfahren nach Anspruch 8, bei dem in Fällen, in denen eine Brust einer untersuchten Person abgebildet wird, die hexagonalförmigen Pixel abgeflacht sind, und die Bildgebung in der Weise erfolgt, dass die Brust so angeordnet ist, dass die kürzere Pixelbreite entlang einer Richtung ausgerichtet ist von einer Brustwandseite in Richtung der Spitze der Brust.

**10.** Verfahren nach einem der Ansprüche 8 bis 9, bei dem die Bildgebung mit einem Strahlungsdetektorelement ausgeführt wird, in welchem die hexagonalförmigen Pixel derart abgeflacht sind, dass eine von drei Diagonalen, die durch die Mitte der Pixel verlaufen, kürzer ist als die zwei anderen Diagonalen, und die zwei anderen Diagonalen gleiche Länge haben.

**11.** Verfahren nach einem der Ansprüche 7 bis 10, bei dem die Interpolationsverarbeitung als erstes in der Richtung von einer horizontalen Richtung und einer vertikalen Richtung ausgeführt wird, in der die ersten Bilddaten einen kürzeren Pixelanordnungs-Mittenabstand aufweisen, und dann die Interpolationsverarbeitung in der anderen Richtung erfolgt.

**12.** Verfahren nach einem der Ansprüche 1 bis 11, weiterhin umfassend das Ausgeben eines Bilds basierend auf den zweiten Bilddaten.

**Revendications**

1. Dispositif d'imagerie radiographique, comprenant :

   un élément de détection de rayonnement incluant une pluralité de pixels de forme hexagonale et de même taille, qui détectent un rayonnement et sont disposés suivant un motif en nid d'abeille, et
   une section de conversion de densité de pixels qui réalise un traitement d'interpolation de telle sorte que des premières données d'image obtenues à partir de l'élément de détection de rayonnement sont converties en secondes données d'image représentant une image d'une pluralité de pixels disposés suivant un motif de grille carrée,
   dans lequel la conversion de pixels est réalisée de telle sorte que la formule suivante (1) est satisfaite dans le dispositif d'imagerie radiographique :

$$d2max \leq d1max \leq \sqrt{(2 \times S1)} \qquad \text{Formule 1}$$

   où d1max indique la longueur d'une diagonale la plus longue des pixels de forme hexagonale ; S1 indique l'aire des pixels de forme hexagonale, et d2max indique la longueur d'une diagonale du treillis carré des secondes données d'image.

2. Dispositif d'imagerie radiographique selon la revendication 1, dans lequel les pixels de forme hexagonale sont formés avec des formes hexagonales aplaties.

3. Dispositif d'imagerie radiographique selon la revendication 2, dans lequel dans les cas où le dispositif d'imagerie radiographique est employé comme appareil de mammographie pour imager un sein d'un sujet d'examen, les pixels de forme hexagonale sont formés de manière aplatie, de sorte qu'une longueur dans une direction de profondeur allant d'un côté paroi thoracique jusqu'à la pointe du sein est plus courte qu'une largeur dans une direction orthogonale à cette direction.

4. Dispositif d'imagerie radiographique selon la revendication 2 ou 3, dans lequel les pixels de forme hexagonale sont formés de manière aplatie, de telle sorte qu'une diagonale, parmi 3 diagonales traversant le centre des pixels, est plus courte que les 2 autres diagonales, et que les 2 autres diagonales sont de même longueur entre elles.

5. Dispositif d'imagerie radiographique selon l'une quelconque des revendications 1 à 4, dans lequel la section de conversion de densité de pixels réalise un traitement d'interpolation tout d'abord dans la direction parmi une direction horizontale et une direction verticale dans les première données d'image, qui présente un pas de disposition de pixels plus court, puis réalise le traitement d'interpolation dans l'autre direction.

6. Dispositif d'imagerie radiographique selon l'une quelconque des revendications 1 à 5, comprenant en outre :

   une source de rayonnement qui émet un rayonnement, et
   un dispositif de sortie d'image qui produit une image sur la base des secondes données d'image.

7. Procédé d'imagerie radiographique, comprenant :

   la détection de premières données d'image à l'aide de l'élément de détection de rayonnement incluant une pluralité de pixels de forme hexagonale et de même taille, qui détectent un rayonnement et sont disposés suivant un motif en nid d'abeille, et
   la réalisation d'un traitement d'interpolation de telle sorte que les premières données d'image sont converties en secondes données d'image représentant une image d'une pluralité de pixels disposés suivant un motif de grille carrée,
   dans lequel dans le procédé d'imagerie radiographique, les pixels hexagonaux et les pixels dans la grille carrée sont configurés de telle sorte que la formule suivante (1) est satisfaite :

$$d2max \leq d1max \leq \sqrt{(2 \times S1)} \qquad \text{Formule 1}$$

où d1max indique la longueur d'une diagonale la plus longue des pixels de forme hexagonale ; S1 indique l'aire des pixels de forme hexagonale, et d2max indique la longueur d'une diagonale du treillis carré des secondes données d'image.

8. Procédé d'imagerie radiographique selon la revendication 7, dans lequel les pixels de forme hexagonale sont formés avec des formes hexagonales aplaties.

9. Procédé d'imagerie radiographique selon la revendication 8, dans lequel dans les cas où un sein d'un sujet d'examen est imagé, les pixels de forme hexagonale sont formés de manière aplatie, et l'imagerie est réalisée avec le sein disposé de telle sorte qu'une largeur de pixels plus courte est alignée le long d'une direction allant d'un côté paroi thoracique jusqu'à la pointe du sein.

10. Procédé d'imagerie radiographique selon la revendication 8 ou 9, dans lequel l'imagerie est réalisée avec un élément de détection de rayonnement dans lequel les pixels de forme hexagonale sont formés de manière aplatie, de telle sorte qu'une diagonale, parmi 3 diagonales traversant le centre des pixels, est plus courte que les 2 autres diagonales, et que les 2 autres diagonales sont de même longueur entre elles.

11. Procédé d'imagerie radiographique selon l'une quelconque des revendications 7 à 10, dans lequel le traitement d'interpolation est tout d'abord réalisé dans la direction parmi une direction horizontale et une direction verticale dans les premières données d'image, qui présente un pas de disposition de pixels plus court, puis le traitement d'interpolation est réalisé dans l'autre direction.

12. Procédé d'imagerie radiographique selon l'une quelconque des revendications 7 à 11, comprenant en outre la sortie d'une image sur la base des secondes données d'image.

# FIG.1

100

## IMAGING DEVICE 41

### 46
- 46A CPU
- 46B ROM
- 46C RAM
- 46D STORAGE SECTION

24 RADIATION IRRADIATION SECTION
RADIATION SOURCE 30

42 RADIATION DETECTOR

44 OPERATION PANEL

47 DISPLAY

48 COMMUNICATION I/F SECTION

## IMAGE PROCESSING APPARATUS 50

60 CPU

62 ROM

64 RAM

66 HDD

72 COMMUNICATION I/F SECTION

74 IMAGE SIGNAL OUTPUT SECTION

68 DISPLAY DRIVER

52 DISPLAY

70 OPERATION INPUT DETECTION SECTION

54 OPERATION INPUT SECTION

BUS

## DISPLAY DEVICE 80

58

80A DISPLAY SECTION

56

## MANAGEMENT SERVER 57

STORAGE SECTION 57A

EP 2 742 863 B1

# FIG.2

FIG.3

20

FIG.4

# FIG.5

(1)

(2)

# FIG.6

$$d2max \leq d1max \leq \sqrt{(2 \times S1)}$$ ( 1 )

| SQUARE CORRESPONDING TO S1 | | | HEXAGONAL SHAPE | | | SQUARE AFTER CONVERSION (MINIMUM OUTPUT PIXEL SIZE) | | |
|---|---|---|---|---|---|---|---|---|
| S0 | PP0 | d0max | S1 | PP1 (x) | d1max | S2 | PP2 | d2max |
| 4871.4 | 69.8 | 98.7 | 4871.4 | 75 | 86.6 | 2500 | 50 | 70.7 |

$$S0 = S1 \quad \sqrt{S1} \quad \sqrt{(2 \times S1)}$$

( 2 )

CASES OF FLATTENED HEXAGONAL SHAPES

| S0 | PP0 | d0max | S1 | PP1 (x) | d1 (y) | FLATTENING RATIO | d1max |
|---|---|---|---|---|---|---|---|
| 8660.3 | 93.1 | 131.6 | 8660.3 | 100 | 115.5 | 100% | 115.5 |
| 4950.0 | 70.4 | 99.5 | 4950.0 | 100 | 66.0 | 63% | 105.3 |
| 5625.0 | 75.0 | 106.1 | 5625.0 | 100 | 75.0 | 70% | 106.8 |

# FIG.7

$$\sqrt{S1} \leq d2max \leq d1max$$ ( 1 )

| REGULAR HEXAGONAL SHAPE | | | SQUARE AFTER CONVERSION | | | |
|---|---|---|---|---|---|---|
| S1 | PP1 (x) | d1max | S2 | PP2 | d2max | $\sqrt{S1}$ |
| 4489.5 | 72 | 83.1 | 2500 | 50 | 70.7 | 67.0 |

( 2 )

| S1 | PP1 (x) | d1max | S2 | PP2 | d2max | $\sqrt{S1}$ |
|---|---|---|---|---|---|---|
| 4243.5 | 70 | 80.8 | 4225 | 65 | 91.9 | 65.1 |
| 4243.5 | 70 | 80.8 | 3600 | 60 | 84.9 | 65.1 |
| 4243.5 | 70 | 80.8 | 3025 | 55 | 77.8 | 65.1 |
| 4243.5 | 70 | 80.8 | 2500 | 50 | 70.7 | 65.1 |
| 4243.5 | 70 | 80.8 | 2025 | 45 | 63.6 | 65.1 |

## FIG.8A

FIG.8B

# FIG.9

EP 2 742 863 B1

## REFERENCES CITED IN THE DESCRIPTION

**Patent documents cited in the description**

- JP 2003255049 A
- JP 2006029839 A

- JP 2000244733 A

**Non-patent literature cited in the description**

- **VAN DE VILLE D et al.** Image resampling between orthogonal and hexagonal lattices'', International conference on image processing (ICIP). IEEE, 22 September 2002, vol. 3, 389-392